# EUROPÄISCHE PATENTSCHRIFT

(11) **EP 0 485 805 B1**
(45) Veröffentlichungstag und Bekanntmachung des Hinweises auf die Patenterteilung: **27.12.1995**
(21) Anmeldenummer: 91118476.0
(22) Anmeldetag: 30.10.1991
(51) Int. Cl.: G01V 3/10, A61B 5/06

(54) **Suchgerät für Zahnimplantate**
Detection apparatus for tooth implants
Appareil de détection pour implants dentaires

(30) Priorität: 15.11.1990 DE 9015639 U
(43) Veröffentlichungstag der Anmeldung: 20.05.1992
(73) Patentinhaber: FRIATEC AG KERAMIK- UND KUNSTSTOFFWERKE, D-68229 Mannheim (DE)
(72) Erfinder: Weber, Heiner, Prof. Dr. med. dent. Zahnprothetik, W-7400 Tübingen (DE); Benzing, Ulrike R., Dipl.-Ing. (FH) Zahnprothetik, W-7400 Tübingen (DE)
(74) Vertreter: Klose, Hans, Dipl.-Phys.

(56) Entgegenhaltungen:
- EP-A- 0 320 623
- WO-A-86/02539
- DE-A- 3 531 389
- FR-A- 2 635 259
- US-A- 4 526 177

## Beschreibung

Die Erfindung betrifft die Verwendung eines Suchgeräts zum Auffinden von Metall enthaltenden Zahnimplantaten.

Aus der US-A-4 526 177 ist ein Suchgerät mit einer rohrförmigen Halterung bekannt, an deren einem Ende ein Ferritkern angeordnet ist. Eine Suchspule ist um diesen Ferritkern gewickelt und mit diesem zusammen in die rohrförmige Halterung eingesetzt, wobei das vordere freie Ende mittels einer Kappe oder eines Stopfens aus Epoxidharz oder dergleichen abgeschlossen ist.

Aus dem anderen Ende der rohrförmigen Halterung ist eine Kabel herausgeführt, über welches die elektrische Verbindung mit einem externen Steuergerät erfolgt. Der in den inneren Hohlraum der Halterung einzusetzende Ferritkern bedingt einen vergleichsweise großen Außendurchmesser der rohrförmigen Halterung. welche zudem eine hinreichend große Wanddicke aus Gründen der Stabilität aufweisen muß. Der Ferritkern erfordert ein zusätzliches Gewicht und eine genaue Markierung der Fundstelle eines Metall enthaltenden Körpers kann nicht ohne weiteres erfolgen.

Aus der WO-A-86/02 539 ist eine Vorrichtung zur Lokalisierung von metallischen Gegenständen im menschlichen oder tierischen Körper bekannt. welche eine ringförmige, in einen Schalenkern eingebettete Suchspule enthält. Im Hinblick auf die relativ geringe Wirkungstiefe bzw. Eindringtiefe einer flachen Ringspule wird in der genannten WO-A daher eine bevorzugte stiftförmige Ausbildung der Sonde mit einem Ferritstab hervorgehoben, auf welchen die Suchspule gewickelt ist. Weder bei Ausbildung des Kerns als Feritstab noch bei Ausbildung als Schalenkern kann eine genaue Markierung der Fundstelle eines metallischen Körpers vorgenommen werden.

Ferner ist aus der FR-A-26 35 259 ein Suchgerät mit einer auf einen Ferritstab gewickelten Suchspule bekannt. Das Suchgerät enthält sowohl eine Elektronik zur Signalauswertung als auch eine Batterie zur Stromversorgung. Das Suchgerät weist vergleichsweise große Abmessungen und ein nicht unerhebliches Gewicht auf und kann daher in der Zahnheilkunde nicht ohne weiteres zum Einsatz gelangen.

Schließlich ist aus der EP-A-320 623 eine Vorrichtung zur Lageortung eines Katheders oder einer Sonde in einem Organ eines Lebewesens bekannt, wobei ein Metalldetektor zur Erzeugung eines elektromagnetischen Feldes vorgesehen ist. Der Metalldetektor ist als ein vergleichsweise großes Handgerät, ähnlich einer Taschenlampe, ausgebildet, dessen zylindrischer Kopfteil eine Suchspule enthält. In einem Griffteil sind ein Schalter, eine Auswerteelektronik, eine Lampe oder ein kleiner Lautsprecher vorgesehen. Die Suchspule ist in einem Schalenkern angeordnet und im Hinblick auf die vergleichsweise großen Abmessungen kann dieses Suchgerät nicht ohne weiteres in der Zahnheilkunde zum Auffinden und zur exakten Positionsbestimmung eines Implantats verwendet werden.

Bekanntlich gelangen in der Zahnheilkunde Dentalimplantate zum Einsatz, welche im Kiefer verankerbar sind und auf welchen Kronen, Brücken oder Stege befestigt werden können. So ist aus der DE-A-35 31 389 ein enossales Dentalimplantat bekannt, welches "zweiphasig" ausgebildet ist. Derartige Implantate weisen regelmäßig einen Durchmesser von 3,3 bis 4 Milimeter auf und nach dem Einbringen in den Kiefer wird die Wunde zunächst wieder mit Zahnfleisch bedeckt. Während der Einheilungsphase ist das Implantat mit einer flachen Titanschraube verschlossen. Nach einer Einheilungszeit des Implantats von etwa drei bis vier Monaten muß das Zahnfleisch über dem Implantat entfernt werden, damit eine Suprakonstuktion mit dem Implantat verbunden werden kann. Oftmals kann die genannte Titanschraube und/oder das Implantat vom Arzt unter dem Zahnfleisch erfühlt werden, welches dann mittels einer Elektroschlinge entfernt wird, um die Verschlußschraube freizulegen. Diese Methode versagt jedoch, wenn das Implantat sehr tief liegt, bereits Knochen über das Implantat gewachsen ist oder das Implantat mit Hydroxylapatit überdeckt wurde. In derart gelagerten Fällen muß der Knochen an der vermuteten Stelle des Implantats unter Umständen großräumig freigelegt werden. Die hiermit verbundenen Nachteile und Risiken in der Praxis liegen auf der Hand.

Der Erfindung liegt daher die Aufgabe zugrunde, das Suchgerät zum Auffinden von Metall enthaltenden und schleimhautbedeckten Implantaten auszubilden, wobei mit geringem Gewicht eine einfache Markierung der Fundstelle ermöglicht werden soll.

Diese Aufgabe wird gemäß den im Patentanspruch 1 angegebenen Merkmalen gelöst.

Durch die vorgeschlagene Verwendung des Suchgeräts wird eine sehr exakte Ortung des Zahnimplantats oder einer Verschluß-Schraube oder dergleichen aus metallischen Werkstoff ermöglicht. Infolge der kernfreien und freigewickelten Ausbildung der Suchspule werden ein kompakter Aufbau, ein geringes Gewicht sowie eine einfache Handhabbarkeit erreicht, welche in der Zahnheilkunde wichtige Einsatzkriterien darstellen. Die stabförmige Halterung enthält den die Suchspule vollständig umgebenden Mantel und bildet mit diesen eine integrale Einheit. Die Halterung mit dem Mantel der Suchspule besteht aus einem temperaturbeständigen Kunststoff, um problemlos die Sterilisation im Autoklav zu ermöglichen. Aufgrund der ringförmigen Ausbildung der Suchspule und der zentralen Öffnung des Mantels wird eine einfache Markierung der Fundstelle ermöglicht, so daß eine großräumige Freilegung des Knochens vermieden wird. Durch die kernfreie, freigewickelte Ausbildung der Suchspule wird eine Miniuatisierung erreicht und die Suchspule kann problemlos in einen Zahnzwischenraum zwecks Auffinden einer Verschlußschraube des Dentalimplantats oder dergleichen eingeführt werden. Die Suchspule ist in den Mantel der stabförmigen Halterung zweckmäßig eingegossen und somit leicht und flexibel zu handhaben, während die elektrische Stromversorgung und Auswerteelektronik in einem separaten kleinen Schaltgerät untergebracht sind, wobei mittels eines flexiblen elektrischen Kabels die elektrische Verbindung hergestellt wird.

Besondere Ausgestaltungen und Weiterbildungen der Erfindung sind in den abhängigen Ansprüchen angegeben.

Nachfolgend wird die Erfindung anhand des in der Zeichnung dargestellten Ausführungsbeispiels näher erläutert.

Es zeigen:
- Fig. 1: schematisch eine Ansicht des Suchgerätes,
- Fig. 2: den Schaltplan der Elektronik.

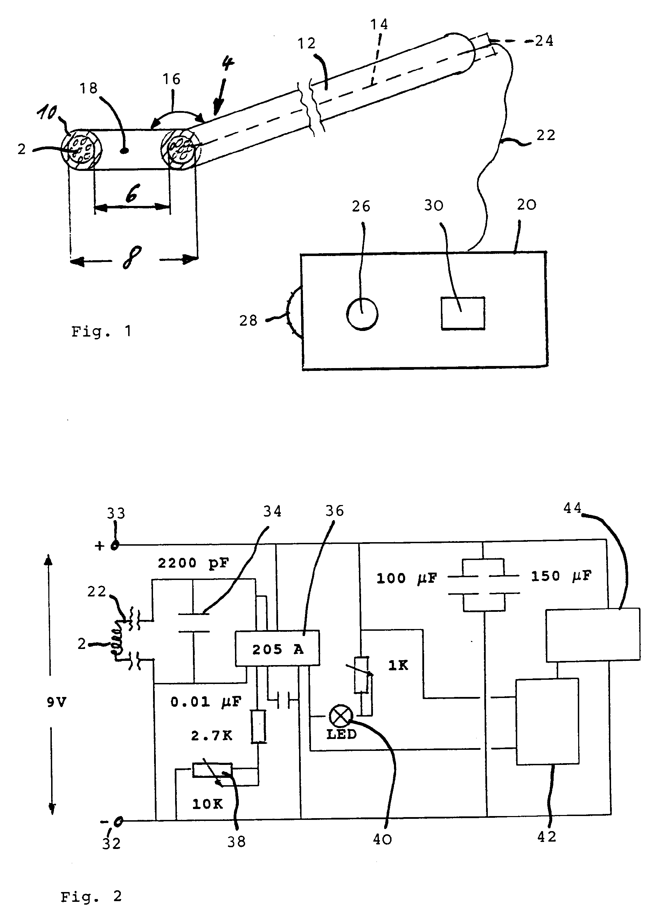

Fig. 1 zeigt vergrößert eine Suchspule 2, welche in einer Kunststoffhalterung 4 eingebettet ist. Die Suchspule 2 ist erfindungsgemäß ringförmig ausgebildet. Die ringförmige Suchspule 2 enthält innen eine zentrale Öffnung. Der Innendurchmesser 6 ist bevorzugt zwischen 4 und 6 mm groß und beträgt insbesondere im wesentlichen 5 mm. Der Außendurchmesser 8 der ringförmigen Suchspule 2 liegt bevorzugt im Bereich zwischen 7 und 9 mm und beträgt insbesondere 8 mm. Wie ersichtlich, ist die Suchspule allseitig von einem Mantel 10 der Kunststoffhalterung umgeben, wobei die Wanddicke ca. 1 mm beträgt. Die Halterung und somit der Mantel 10 bestehen aus einem temperaturbeständigen Kunststoff, welcher in einfacher Weise die Sterilisation durch Erwärmung im Autoklav oder dergleichen zuläßt. Die Kunststoffhalterung umschließt allseitig die ringförmige Spule, welche frei gewickelt ist und/oder keinen magnetisch wirksamen Kern aufweist. Durch die in der Mitte befindliche Öffnung kann, insbesondere mittels einer Sonde, in dem Zahnfleisch eine Markierung angebracht werden kann.

Die Halterung 4 enthält ferner einen Griff 12, welcher zusammen mit der Suchspule 2 und dem Mantel 10 eine integrale Baueinheit bildet. Durch den Griff 12 sind elektrische Anschlußleitungen 14 der Suchspule 2 nach außen geführt. Der Griff 12 kann in zweckmäßiger Weise bezüglich der durch die Suchspule 2 verlaufenden Ebene um einen stumpfen Winkel 16 geneigt angeordnet sein, damit die Suchspule 2 möglichst ungehindert in die Nähe des Implantates gebracht werden kann. Entsprechend der empirisch ermittelten Windungszahl der Suchspule kann diese eine Reichweite bis zu einer Tiefe von ca. 6 mm aufweisen. Die ringförmige Suchspule 2 weist konzentrisch im Mantel 10 eine zentrale Öffnung 18 auf, um nach dem Auffinden des Implantats oder der genannten Verschlußschraube in einfacher Weise eine Markierung auf dem Zahnfleisch vorsehen zu können. Der stumpfe Winkel 16 zwischen der Längsachse des Griffes 12 und der durch die Spule verlaufenden Ebene ist definiert vorgegeben. Der Mantel 10 und der Griff 12 sind aus dem gleichen Kunststoff einstückig gefertigt und ergeben eine dichte und den Anforderungen der Hygiene gerecht werdende Ummantelung. Die erfindungsgemäß in die Kunststoffhalterung eingebettete Suchspule 2 ist aus Kupferdraht frei gewickelt. Diese erfindungsgemäße, frei gewickelte Suchspule, welche in zweckmäßiger Weise keinen Kern enthält, erfordert ein minimales Volumen und weist ferner ein sehr geringes Gewicht auf. Hierdurch wird in besonders zweckmäßiger Weise die Handhabung des Suchgerätes erleichtert.

Die Elektronik ist in einem externen Steuergerät 20 angeordnet, wobei mittels eines bevorzugt flexiblen Kabels 22 die elektrische Verbindung mit der Suchspule 2 hergestellt wird. Das Kabel 22 kann direkt an den Griff 12 angeformt sein oder aber mittels einer hier nur schematisch angedeuteten Steckverbindung 24 verbunden sein. Das Steuergerät 20 enthält innen eine Schaltplatine sowie eine Stromversorgung, wobei bedarfsweise auch ein wiederaufladbarer Akku vorgesehen werden kann. Wie schematisch angedeutet, enthält das Steuergerät ferner außen eine Leuchtanzeige 26, insbesondere in Form einer Leuchtdiode (LED), ein kleines Stellrad 28 zur Empfindlichkeitsregelung sowie einen Schalter 30 zum Ein- bzw. Ausschalten. Auch das Steuergerät weist kompakte Abmessungen von wenigen Zentimetern auf und kann ohne weiteres in die Hand genommen und getragen werden.

Fig. 2 zeigt den Schaltplan der im Steuergerät 20 auf einer Platine angeordneten Elektronik. Zur Stromversorgung an den Klemmen 32, 33 kann eine handelsübliche Batterie oder ein Akkumulator von 9 V angelegt werden. Selbstverständlich kann im Rahmen der Erfindung über ein Netzgerät die Stromversorgung erfolgen. Die Suchspule 2 ist hier der Einfachheit halber unmittelbar neben dem Kondensator 34 eines LC-Oszilators dargestellt, doch ist gemäß obigen Ausführungen die Suchspule 2 extern angeordnet und mittels des Kabels 22 mit dem Steuergerät verbunden. Die Suchspule 2 und der Kondensator 34 sind an einen integrierten Schaltkreis (IC) 36 vom Typ C 205 A der Firma Siemens AG, München, angeschlossen. Mittels eines Wendelpotentiometers 38 wird über das oben erwähnte kleine Stellrad die Empfindlichkeit eingestellt. Hierbei kann es sich um ein Zehngang-Wendelpotentiometer handeln, das einen Gesamtwiderstand von beispielsweise 10 Kiloohm aufweist. Im Schaltkreis 36 sind eine Leuchtdiode 40 sowie eine Relais 42 nachgeschaltet, über welches ein Summer 44 schaltbar ist.

Wenn die Suchspule 2 in die Nähe eines Metallteiles gebracht wird bzw. im wesentlichen senkrecht über einem Implantat und/oder einer Verstellschraube aus Metall, und zwar insbesondere aus Titan, gebracht ist, bricht die Schwingung des hochfrequenten Schwingkreises völlig zusammen. Durch das Aussetzen der Schwingung wird in den IC-Schaltkreis 36 die Ausgangsspannung derart erhöht, daß die nachgeschaltete Leuchtdiode 40 aufleuchten kann und das Relais 42 schließt mit der Folge, daß der Summer 44 ertönt. Wird die Suchspule 2 von dem Metallteil entfernt, so setzt die Schwingung wieder ein, so daß der Summer 44 verstummt und die Leuchtdiode 40, welche der vorstehend erwähnten Leuchtanzeige des Steuergerates entspricht, erlischt.

### Bezugszeichen

- 2: Suchspule
- 4: Kunststoffhalterung
- 6: Innendurchmesser
- 8: Außendurchmesser
- 10: Mantel
- 12: Griff/Stab
- 14: Anschlußleitung
- 16: Winkel
- 18: Öffnung in 10
- 20: Steuergerät
- 22: Kabel
- 24: Steckverbindung
- 26: Leuchtanzeige
- 28: Stellrad
- 30: Schalter
- 32, 33: Anschlußleitung
- 34: Kondensator
- 36: integrierter Schaltkreis
- 38: Potentiometer
- 40: Leuchtdiode
- 42: Relais
- 44: Summer

## Patentansprüche

1. Verwendung eines Suchgeräts zum Auffinden von Metall enthaltenden Zahnimplantaten, wobei das Suchgerät eine Halterung (4) mit einer eingebetteten Suchspule (2) aufweist, welche mit einem externen Steuergerät (20) elektrisch verbunden ist und welche ringförmig, kernfrei und freigewickelt ausgebildet ist, und wobei ein die Suchspule (2) einschließender Mantel (10) der Halterung (4) eine zentrale Öffnung (18) aufweist.

2. Verwendung eines Suchgeräts nach Anspruch 1, dadurch gekennzeichnet, daß der Mantel (10) mit der Suchspule (2) zu einem Griff (12) der Halterung (4) in einem stumpfen Winkel (16) angeordnet ist.

3. Verwendung eines Suchgeräts nach Anspruch 2, dadurch gekennzeichnet, daß durch den Griff (12) die elektrischen Anschlußleitungen (14) der Suchspule (2) nach außen geführt sind und daß am freien Ende des Griffs (12) eine Steckverbindung (24) angebracht ist, an welcher ein mit dem Steuergerät (20) verbundenes flexibles Kabel (22) anschließbar ist.

4. Verwendung eines Suchgeräts nach einem der Ansprüche 1 bis 3, dadurch gekennzeichnet, daß die Suchspule (2) einen Außendurchmesser (8) aufweist, welcher zwischen 7 und 9 mm groß ist und insbesondere 8 mm beträgt.

5. Verwendung eines Suchgeräts nach einem der Ansprüche 1 bis 4, dadurch gekennzeichnet, daß die Suchspule (2) einen Innendurchmesser (6) aufweist, welcher zwischen 4 und 6 mm groß ist und insbesondere 5 mm beträgt.

## Claims

1. Use of a detection apparatus to locate dental implants containing metal, in which the detection apparatus has a holder (4) with an embedded search coil (2) which is electrically connected to an external controller (20) and is annular, coreless and freely wound, and in which a casing (10) of the holder (4) enclosing the search coil (2) has a central opening (18).

2. Use of a detection apparatus according to claim 1, characterised in that the casing (10) with the search coil (2) is arranged at an acute angle (16) to a handle (12) of the holder (4).

3. Use of a detection apparatus according to claim 2, characterised in that the electrical connecting leads (14) of the search coil (2) are led out through the handle (12) and that a connector (24) to which a flexible cable (22) connected to the controller (20) can be connected is fitted to the free end of the handle (12).

4. Use of a detection apparatus according to one of claims 1 to 3, characterised in that the search coil (2) has an external diameter (8) of between 7 and 9 mm, in particular 8 mm.

5. Use of a detection apparatus according to one of claims 1 to 4, characterised in that the search coil (2) has an internal diameter (6) of between 4 and 6 mm, in particular 5 mm.

## Revendications

1. Utilisation d'un appareil de détection aux fins de repérer des implants dentaires contenant du métal, l'appareil de détection présentant un support (4) avec une bobine de détection (2) noyée dans celui-ci, laquelle est relié électriquement à un appareil de commande externe (20) et est conformée de manière annulaire, sans noyau et à enroulement libre, et un manteau (10) du support (4) enserrant la bobine de détection (2) présentant une ouverture centrale (18).

2. Utilisation d'un appareil de détection selon la Revendication 1, ***caractérisée en ce que*** le manteau (10), avec la bobine de détection (2), est placée selon un angle obtus (16) par rapport à une poignée (12) du support (4).

3. Utilisation d'un appareil de détection selon la Revendication 2, ***caractérisée en ce que*** les conduites de raccordement électrique (14) de la bobine de détection (2) passent vers l'extérieur à travers la poignée (12), et ***en ce qu'***une prise (24) est prévue à l'extrémité libre de la poignée (12), prise sur laquelle se branche un câble flexible (22) relié à l'appareil de commande (20).

4. Utilisation d'un appareil de détection selon l'une des Revendications 1 à 3, ***caractérisée en ce que*** la bobine de détection (2) présente un diamètre extérieur (8) qui est compris entre 7 et 9 mm, et qui vaut en particulier 8 mm.

5. Utilisation d'un appareil de détection selon l'une des Revendications 1 à 4, ***caractérisée en ce que*** la bobine de détection (2) présente un diamètre intérieur (6) qui est compris entre 4 et 6 mm, et qui vaut en particulier 5 mm.
